(19) Europäisches Patentamt
European Patent Office
Office européen des brevets

(11) EP 1 913 964 A2

(12) # EUROPEAN PATENT APPLICATION
published in accordance with Art. 153(4) EPC

(43) Date of publication:
**23.04.2008 Bulletin 2008/17**

(21) Application number: **06782579.4**

(22) Date of filing: **09.08.2006**

(51) Int Cl.:
***A61M 1/14*** *(2006.01)*
***A61M 1/18*** *(2006.01)*
***B01D 63/02*** *(2006.01)*
***B01D 65/02*** *(2006.01)*
***B01D 71/44*** *(2006.01)*
***B01D 71/68*** *(2006.01)*
***D01F 6/94*** *(2006.01)*

(86) International application number:
**PCT/JP2006/315763**

(87) International publication number:
**WO 2007/018242 (15.02.2007 Gazette 2007/07)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.08.2005 JP 2005232154**

(71) Applicant: **Asahi Kasei Kuraray Medical Co., Ltd.**
**Tokyo 101-8482 (JP)**

(72) Inventors:
- **KOMURA, Ryo**
**Tokyo1008440 (JP)**
- **KOIZUMI, Toshinori**
**Tokyo 1008440 (JP)**

(74) Representative: **Weber, Thomas et al**
**Patentanwälte**
**von Kreisler-Selting-Werner,**
**Bahnhofsvorplatz 1 (Deichmannhaus)**
**50667 Köln (DE)**

(54) **HOLLOW FIBER MEMBRANE TYPE BLOOD PURIFIER**

(57) A hollow fiber membrane type blood purification device packed with hollow fiber membranes comprising a polysulfone resin and polyvinylpyrrolildone. Even when sterilized by irradiation with a radiation, the hollow fiber membrane type blood purification device is less apt to suffer polyvinylpyrrolidone elution and can be inhibited from decreasing in blood compatibility. The purification device is of the type called semi-dry, and is lightweight and has excellent handleability.

The hollow fiber membrane type blood purification device comprises a bundle of hollow fiber membranes comprising a polysulfone resin and polyvinylpyrrolidone, a container packed therewith, and a potting agent disposed between each bundle end and the container to hold the bundle and thereby form a hollow-fiber-membrane inside chamber and a hollow-fiber-membrane outside chamber. The purification device has fluid inlet and outlet ports connected to the hollow-fiber-membrane inside chamber and fluid inlet and outlet ports connected to the hollow-fiber-membrane outside chamber. The hollow fiber membrane type blood purification device is **characterized in that** the hollow fiber membranes have a adhesion rate of a radical trapping agent of 80-300% based on the dry weight of the hollow fiber membranes, have a water content of not less than 40% and less than 100%, and have been sterilized with a radiation.

EP 1 913 964 A2

## Description

TECHNICAL FIELD

The present invention relates to a hollow fiber membrane type blood purification device used for extracorporeal circulation type blood purification therapy. More particularly, the present invention relates to a hollow fiber membrane type blood purification device so-called radiation-sterilized semi-dry type which has a reduced weight and excellent handling properties, allows elution of only a small amount of hydrophilic polymer, and exhibits excellent blood compatibility.

BACKGROUND ART

Various hollow fiber membrane type blood purification devices have been developed according to extracorporeal circulation type blood purification therapy such as hemodialysis, hemofiltration, plasma separation, and plasma fractionation up to now. Hollow fiber membrane type blood purification devices with improved safety and performance have been put to practical use.
Hollow fiber membranes used for such hollow fiber membrane type blood purification devices are mainly formed of a cellulose polymer or a synthetic polymer. In recent years, a polysulfone-based resin among the latter has been widely used as the membrane material. A polysulfone-based resin exhibits excellent biocompatibility and excellent resistance to radiation, heat, and chemicals such as acids and alkalis. On the other hand, a polysulfone-based resin is hydrophobic and lacks compatibility to blood. Therefore, a hydrophilic polymer which has few stimuli to blood, such as polyvinylpyrrolidone or polyethylene glycol, is added to a polysulfone-based resin as a hydrophilic agent.
Hollow fiber membrane type blood purification devices are roughly classified as a wet type, in which the insides of the hollows of the hollow fiber membranes and the space between the hollow fiber membranes and a container are filled with an aqueous medium, and a non-wet type in which an aqueous medium is not filled.
The latter may be further classified as a dry type in which the membranes have a low water content of about several percent and a semi-dry type (also may be referred to as "half-wet type") in which the membranes are moderately wetted with water, a wetting agent, or the like. The dry type and semi-dry type hollow fiber membrane type blood purification devices have a light product weight as compared with the wet type hollow fiber membrane blood purification devices, and are hard to freeze at a low temperature. Therefore, the dry type and semi-dry type hollow fiber membrane type blood purification devices have a product form excellent in especially distribution of transportation and storage.
These hollow fiber membrane type blood purification devices must be completely sterilized before use. Irradiation has been employed as a suitable sterilization method because irradiation can sterilize a material to be sterilized in a hermetically packaging state while achieving a high sterilization effect.
On the other hand, a hydrophilic polymer or the like contained in the membrane used for the hollow fiber membrane type blood purification device is denatured and deteriorates due to irradiation. As a result, it is known that the eluted amount of hydrophilic polymer from the hollow fiber membranes increases, whereby blood compatibility deteriorates. In order to prevent such a problem, Patent Document 1, for example, discloses a blood purification device in which hollow fiber membranes have a water content equal to or higher than the saturation water content. Such a wet type hollow fiber membrane type blood purification device can reduce deterioration of hollow fiber membrane components caused by irradiation.
However, since the above blood purification device requires that the hollow fiber membranes have a water content equal to or higher than the saturation water content, the weight of the blood purification device inevitably increases. Therefore, an increase in transportation cost and a decrease in handling properties may occur. Moreover, the membranes may break due to an increased impact to the membranes upon dropping, whereby the probability of leakage may also increase. In addition, the hollow fiber membranes or the container may break due to freezing in winter or in cold climates. It can not necessarily be satisfied due to the risk to the patient accompanying the break.
As an example which prevents damage due to irradiation while avoiding the risk of the wetting state equal to or higher than the saturation water content, Patent Document 2 discloses a hollow fiber membrane type blood purification device in which the hollow fiber membranes are formed of a polysulfone-based resin and polyvinylpyrrolidone, and the hollow fiber membranes have been radiation-sterilized in a state in which the water content of the membrane is 5% or less and the relative humidity of the atmosphere around the hollow fiber membranes is 40% or less.
This dry type hollow fiber membrane type blood purification device can solve the problem which occurs in Patent Document 1. However, since the membranes cannot be sufficiently protected from the atmosphere, excited oxygen radicals are produced when radiation is applied to the membranes in the air, whereby the membranes are inevitably chemically modified. As a result, the main chains and side chains of the polymers forming the membranes are cut due to oxidative decomposition, whereby the polymer (particularly hydrophilic polymer) elutes from the hollow fiber membranes. Moreover, blood compatibility may decrease.
Patent Document 3 proposes an example which solves the above problem and protects the membranes from

irradiation. Patent Document 3 discloses a hollow fiber membrane type blood purification device in which the hollow fiber membrane was formed of a polysulfone-based resin and polyvinylpyrrolidone, and the hollow fiber membranes have been radiation-sterilized in a state in which the water content of the hollow fiber membranes is 30 wt% or less of the total amount of polymers and the surface of the hollow fiber membrane is coated with a protective agent in an amount of 20 to 300 wt% of the hollow fiber membrane material.
However, this blood purification device has a problem in that the concentration of the membrane protective agent is high in spite of the low water content of the hollow fiber membranes. Specifically, the surface of the membranes may not be uniformly coated with the membrane protective agent or the membrane protective agent may not uniformly reach the inside of the membranes, whereby irregular coating tends to occur on the membrane surface. Therefore, the blood purification device disclosed in Patent Document 3 may also suffer from an increase in the amount of the hydrophilic polymer eluted from the insufficiently coated portion of the hollow fiber membrane, whereby blood compatibility may decrease.

In addition to the above blood purification devices, some semi-dry type blood purification devices have also been studied. For example, Patent Documents 4 to 6 disclose semi-dry type blood purification devices in which the hollow fiber membranes have a water content of 4% or more or 100 to 600%. However, Patent Documents 4 to 6 merely optimize the water content or control the oxygen concentration to a low level while optimizing the water content. Moreover, Patent Documents 4 to 6 were silent about protecting the membranes certainly using different component. In particular, when the water content exceeds 100%, water droplets adhere to the inner side of the container, whereby the appearance of the product deteriorates.
Patent Document 7 discloses a semi-dry type blood purification device which a 40% glycerol aqueous solution flows therethrough. However, Patent Document 7 is also silent about protecting the membranes, and does not recognize the importance of the adhesion rate and the water content at all.

As described above, a radiation-sterilized semi-dry type hollow fiber membrane type blood purification device which is loaded with hollow fiber membranes formed of a hydrophobic polymer such as polysulfone-based resin and a hydrophilic polymer such as polyvinylpyrrolidone, and said hollow fiber membrane type blood purification device which is suppressed the elution of the hydrophilic polymer from the membranes and the deterioration in blood compatibility has not yet been proposed.

[Patent Document 1] JP-B-S55-23620
[Patent Document 2] JP-A-2000-288085
[Patent Document 3] JP-A-H06-285162
[Patent Document 4] JP-A-2003-245526
[Patent Document 5] JP-A-2001-170167
[Patent Document 6] JP-A-2001-170172
[Patent Document 7] JP-A-2000-196318

## DISCLOSURE OF THE INVENTION

### Problems to be Solved by the Invention

In view of the above-described problems, an object of the present invention is to provide a hollow fiber membrane type blood purification device which is loaded with semi-dry type hollow fiber membranes formed of a polysulfone-based resin and polyvinylpyrrolidone and has reduced weight and excellent handling properties,
wherein the hollow fiber membrane type blood purification device has properties that elution of polyvinylpyrrolidone as the hydrophilic polymer is reduced and a decrease in blood compatibility can be suppressed in spite of being radiation-sterilized.

### Means for Solving the Problems

The inventors of the present invention have conducted extensive studies in order to achieve the above object. As a result, the inventors have found that it is important to protect the hollow fiber membranes from attack by radicals produced during radiation sterilization, therefore, it is very important to use a radical-trapping material and to uniformly cover the surface and the inside of the membranes with the radical trap material. The inventors have also found that it is indispensable to control the amount of the radical-trapping material in the membranes and the water content to a specific relationship. As a result, the inventors have succeeded in significantly suppressing the increase in the amount of elution of the hydrophilic polymer from the hollow fiber membranes and the consequent decrease in blood compatibility in the semi-dry type hollow fiber membrane type blood purification device. These findings led the completion of the present invention.

Specifically, the present invention provides the following inventions.

(1) A hollow fiber membrane type blood purification device comprising a container loaded with a bundle of hollow fiber membranes which are formed of a polysulfone-based resin and polyvinylpyrrolidone, wherein the gaps between each ends of the hollow fiber membrane bundle and the container are held by a potting material to form a hollow fiber membrane inner chamber and a hollow fiber membrane outer chamber, the hollow fiber membrane type blood purification device has fluid inlet and outlet ports connected to the hollow fiber membrane inner chamber and fluid inlet and outlet ports connected to the hollow fiber membrane outer chamber, and the hollow fiber membranes have 80 to 300% of adhesion rate of radical-trapping material to the dry weight of the hollow fiber membranes and not less than 40% and less than 100% of a water content, and the hollow fiber membranes are sterilized by radiation.

(2) The hollow fiber membrane type blood purification device according to (1), wherein the radical-trapping material is a material which inhibits polyvinylpyrrolidone from crosslinking.

(3) The hollow fiber membrane type blood purification device according to (1) or (2), wherein the radical-trapping material is a polyhydric alcohol.

(4) The hollow fiber membrane type blood purification device according to any one of (1) to (3), wherein the radical-trapping material is glycerol.

(5) The hollow fiber membrane type blood purification device according to any one of (1) to (4), wherein the adhesion rate of the radical-trapping material is 100 to 160%.

(6) The hollow fiber membrane type blood purification device according to any one of (1) to (5), wherein the hollow fiber membranes are treated with the radical-trapping material by

a) causing a radical-trapping material solution to pass through the hollow fiber membranes from opening thereof after the gaps between the ends of the hollow fiber membrane bundle and the container have been held by the potting material and before headers have been provided, or
b) causing a radical-trapping material solution to pass through the hollow fiber membranes using fluid inlet and outlet ports connected to the inside of the hollow fiber membranes of the blood purification device after headers respectively having a fluid inlet port and outlet port have been equipped.

(7) The hollow fiber membrane type blood purification device according to any one of (1) to (6), wherein the hollow fiber membranes have been subjected to $\gamma$-ray sterilization in a state in which the oxygen concentration in the hollow fiber membrane type blood purification device is less than 0.1%.

(8) The hollow fiber membrane type blood purification device according to any one of (1) to (7), wherein the amount of polyvinylpyrrolidone eluted from the hollow fiber membranes is not more than 5 mg per 1.5 $m^2$ of the inner surface area of the hollow fiber membranes.

EFFECT OF THE INVENTION

The hollow fiber membrane type blood purification device according to the present invention is called semi-dry type and has a reduced weight and excellent handling properties, exhibits a small amount of elution of polyvinylpyrrolidone even after radiation sterilization, and exhibits excellent blood compatibility. Therefore, the blood purification device according to the present invention may be suitably used for blood purification therapy.

BEST MODE FOR CARRYING OUT THE INVENTION

The present invention is described in detail below.
The hollow fiber membrane type blood purification device according to the present invention comprises a container loaded with a bundle of hollow fiber membranes therein, and the gaps between each end of the bundle and the container are held by a potting material to form a hollow fiber membrane inner chamber and a hollow fiber membrane outer chamber. The hollow fiber membrane type blood purification device has fluid inlet and outlet ports connected to the hollow fiber membrane inner chamber and fluid inlet and outlet ports connected to the hollow fiber membrane outer chamber. For example, commercially available hollow fiber membrane hemodialyzers, hemofilters, hemodiafilters, plasma separators, plasma fractionators, and the like correspond to the hollow fiber membrane type blood purification device

according to the present invention. The hollow fiber membrane type blood purification device having such a structure is suitably used for extracorporeal circulation blood purification therapy.

The term "hollow fiber membrane" used herein is not limited to its shape, size, and fractionation properties. An appropriate hollow fiber membrane may be selected depending on the objective such as hemodialysis, hemofiltration, plasma separation, protein fractionation or the like. As the material for the hollow fiber membrane, a polymer blend membrane formed of a hydrophobic polymer and a hydrophilic polymer is optimum since the pore size is easily controlled during membrane formation and excellent blood compatibility and chemical stability are achieved. In the present invention, a polysulfone-based resin is used as the hydrophobic polymer, and polyvinylpyrrolidone is used as the hydrophilic polymer.

The polysulfone-based resin is aromatic compound and thus exhibits particularly excellent radiation resistance. Moreover, the polysulfone-based resin exhibits excellent resistance to heat and chemical treatment and is also excellent in safety. Therefore, the polysulfone-based resin enables various membrane formation conditions to be employed and allows radiation sterilization. This makes the polysulfone-based resin particularly preferred as the membrane material used for the blood purification device. The term "---based resin" used herein includes not only a homopolymer, but also copolymers with other monomers, and chemically modified analogs thereof.

The term "polysulfone-based resin (hereinafter may be referred to as "PSf")" is a generic name for polymer compounds having a sulfone bond. Examples of the polysulfone-based resin include polysulfone-based polymers of which the repeating units are shown by the following formulas (1), (2), (3), (4), and (5), but not limited thereto. The polysulfone-based resin may be modified polymers in which some of the aromatic rings of these polymers are replaced by a substituent. The aromatic polysulfone-based polymers of which the repeating units are shown by the formulas (1), (2), and (3) are preferable from the viewpoint of industrial availability. Among them, the polysulfone having a chemical structure shown by the formula (1) is particularly preferable. This bisphenol-type polysulfone resin is commercially available as "Udel (registered trademark)" from Solvay Advanced Polymers K.K., for example. This bisphenol-type polysulfone resin is classified depending on the degree of polymerization and the like.

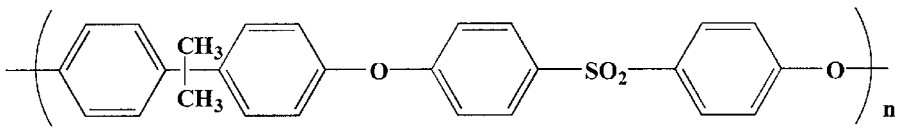

(1)

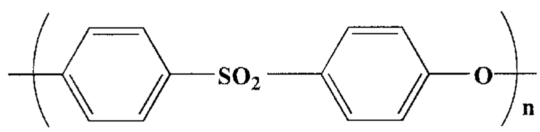

(2)

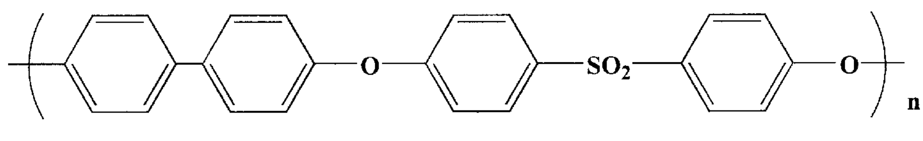

(3)

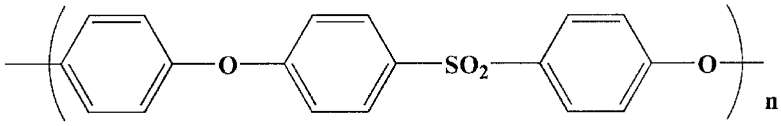

(4)

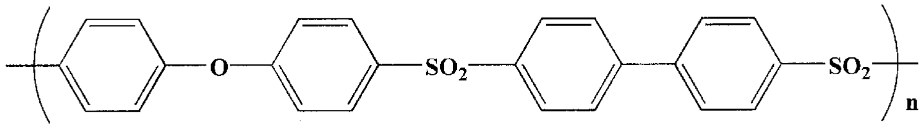

(5)

In the present invention, polyvinylpyrrolidone (hereinafter may be referred to as "PVP") is present in the hollow fiber membrane, and is used to give hydrophilicity to the membrane. PVP is excellent in hydrophilization effect and safety. PVP is classified depending on the molecular weight and the like. For example, PVP K-15, 30, and 90 (manufactured by ISP) are given as commercially available PVP. The molecular weight of the PVP used in the present invention is 10,000 to 2,000,000, and preferably 50,000 to 1,500,000. The PVP content in the membrane is 3 to 20%, and preferably 3 to 10% based on the total amount of the polymer. If the PVP content is 3% or less, since the hydrophilization effect decreases, the resulting hollow fiber membrane may easily cause blood coagulation, whereby blood compatibility may decrease. If the PVP content exceeds 20%, the viscosity of the membrane raw material solution increases to a large extent, thereby resulting in poor productivity.

The hollow fiber membrane formed of the polysulfone-based resin and polyvinylpyrrolidone may be produced utilizing known dry/wet membrane formation technology. Specifically, PSf and PVP are dissolved in a common solvent to prepare a homogenous spinning solution. As examples of the common solvent which dissolves both PSf and PVP, dimethylacetamide (hereinafter referred to as "DMAC"), dimethylsulfoxide, N-methyl-2-pyrrolidone, dimethylformamide, sulfolane, dioxane, and mixtures of two or more of these solvents can be given. Additives such as water may be added to the spinning solution in order to control the pore size.

When forming the hollow fiber membrane, the spinning solution and a hollow-making inner solution which coagulates the spinning solution are simultaneously discharged into the air using a tube-in-orifice spinneret respectively from an orifice and a tube of the spinneret. As the hollow-making inner solution, water or a coagulating solution containing water as the main component may be used. The composition and the like of the hollow-making inner solution may be determined depending on the desired permeability of the hollow fiber membrane. In general, a mixed solution of the solvent used for the spinning solution and water is suitably used. For example, 0 to 60 wt% DMAC aqueous solution or the like may be used.

The spinning solution discharged from the spinneret together with the hollow-making inner solution runs in the air gap and is introduced into and immersed in a coagulation bath which is provided below the spinneret and contains water as a main component to coagulate the spinning solution completely. The coagulated product is subjected to washing step and the like and is winded using a hollow fiber membrane winding machine in a wet state to obtain a hollow fiber membrane bundle. The hollow fiber membrane bundle is then dried. Alternatively, the coagulated product may be dried in a dryer after washing to obtain a hollow fiber membrane bundle. These production method are not particularly limited herein.

A cylindrical hollow fiber membrane is generally used. A hollow fiber membrane in which a fin is provided on the outer surface may also be used. The hollow fiber membrane having a membrane thickness of 1 to 100 $\mu$m, and preferably about 5 to 50 $\mu$m, and an inner diameter of 50 to 500 $\mu$m, and preferably about 100 to 300 $\mu$m may be used. With regard to fractionation properties, a hollow fiber membrane which exhibits high permeability for low-molecular-weight substances to substances having a molecular weight lower than that of albumin is suitably used for dialysis or filtration. A hollow fiber membrane which allows low-molecular-weight protein to pass through but hardly allows high-molecular-weight proteins and immune complexes to pass through is suitably used for protein fractionation. A hollow fiber membrane which allows plasma components to pass through, but does not allow blood cell components to pass through is suitably used for plasma separation.

The hollow fiber membrane type blood purification device may be produced using a known method. For example, the hollow fiber membrane type blood purification device may be produced as follows. Specifically, a hollow fiber membrane bundle is inserted into a tubular container having fluid inlet port and outlet port, and both ends of the bundle are

sealed by injecting a potting material such as polyurethane or the like. After curing, superfluous potting material is cut and removed to open the end face of the hollow fiber membrane bundle, and headers having fluid inlet port and outlet port respectively are attached to the container. According to the above method a hollow fiber membrane bundle is loaded in the container, a hollow fiber membrane inner chamber and a hollow fiber membrane outer chamber are formed in the hollow fiber membrane type blood purification device, whereby the hollow fiber membrane type blood purification device having fluid inlet port and outlet port connected to the hollow fiber membrane inner chamber and fluid inlet port and outlet port connected to the hollow fiber membrane outer chamber can be produced.

The term "radical-trapping material" used in the present invention refers to a liquid component which adheres to the hollow fiber membrane so as to cover the entire surface thereof, and has a function of preventing polyvinylpyrrolidone forming the hollow fiber membrane from deterioration during radiation sterilization (hereinafter also referred to as "irradiation").

The function of the radical-trapping material which prevents deterioration of polyvinylpyrrolidone refers to trapping radicals generated in the membrane due to radiation sterilization or reducing or eliminating the reactivity of radicals by reacting with the radicals.

As typical examples of a compound having such a function, antioxidants such as ascorbic acid, a tocopherol, a polyphenol and the like can be given. More specifically, it is preferable to use vitamins such as vitamin A (derivatives thereof, sodium ascorbate, and palmitol-ascorbate), vitamin C and vitamin E (derivatives thereof and salts such as tocopherol acetate and $\alpha$-tocotrienol), polyhydric alcohols such as glycerol, mannitol, and glycols, saccharideses such as glucose, mannose, xylose, ribose, fructose, and trehalose, fatty acids such as oleic acid, furan fatty acid, thioctic acid, linoleic acid, palmitic acid, and salts and derivatives thereof, and the like.

The radical-trapping material is used in a state in which the radical-trapping material is dissolved or dispersed in a solvent when the raw material is powdery.

When the raw material is oily or liquid, the radical-trapping material is used as is or used in a state in which the radical-trapping material is dissolved or dispersed in a solvent. In the present invention, the term "radical-trapping material" also includes a solution in a state in which a radical-trapping material dissolved or dispersed in a solvent. As the solvent, a physiological solution such as a physiological saline solution, a dialysate, a transfusion, or a buffer solution, water, or an alcohol aqueous solution may be used.

The radical-trapping material adheres to the hollow fiber membrane to cover the entire surface on which polyvinylpyrrolidone is present. Specifically, the radical-trapping material covers the inner surface and the outer surface of the hollow fiber membrane, and the pore surface of the thickness portion of the hollow fiber membrane. Though the pores may be filled with the radical-trapping material, this may cause an increase in weight or leakage. Therefore, it is preferable not to fill with the radical-trapping material.

The adhesion state of the radical-trapping material is not particularly limited. For example, when the radical-trapping material is a liposoluble substance, the radical-trapping material may adhere to the hollow fiber membrane surface through a hydrophobic bond. When the radical-trapping material is a water-soluble substance, the radical-trapping material may be merely held on the membrane surface. Since the radical-trapping material is no longer necessary when using in hospitals, it is preferable that the radical-trapping material adhere to the hollow fiber membrane so that the radical-trapping material is easily washed away and removed from the hollow fiber membrane before use. Therefore, it is preferable that the radical-trapping material merely adhere to the hollow fiber membrane, and it is not in a state in which the majority of the radical-trapping material is strongly bonded to the membrane material through a covalent bond, an ionic bond, or the like, or is insolubilized on the surface of the membrane through crosslinking.

The term "washed away and removed" used herein refers to an operation in which at least 95% of a protective agent which covers the hollow fiber membrane is washed away by a general priming operation before use (e.g., washing operation using several hundreds of milliliters to several liters of a physiological aqueous solution such as a physiological saline solution, dialysate or the like) to reappear the membrane surface formed of the polysulfone-based resin and polyvinylpyrrolidone.

Therefore,

a radical-trapping material is more preferable to simultaneously satisfy such requirements that the radical-trapping material protects from deterioration of polyvinylpyrrolidone, is easily held on the membrane surface due to moderate viscosity, does not form a strong chemical bond with the polysulfone-based resin and polyvinylpyrrolidone, and is easily washed away using a physiological aqueous solution. Specifically, among the above compounds, polyhydric alcohols such as glycerol, mannitol, glycols (e.g., ethylene glycol, diethylene glycol, propylene glycol, and tetraethylene glycol), and polyglycols (e.g., polyethylene glycol) exhibit a high radical-trapping capability per molecule and high solubility in water and physiological solution. Therefore, a polyhydric alcohol aqueous solution easily covers the entire membrane surface and is easily washed away. Accordingly, it is preferable to use a polyhydric alcohol aqueous solution. Among them, an aqueous solution of glycerol or polyethylene glycol is more preferable since glycerol or polyethylene glycol has been used as a pore size retention agent or a surface modifier for blood purification hollow fiber membranes, and an aqueous solution of glycerol is most preferable.

In the present invention, it is necessary that the hollow fiber membrane in the hollow fiber membrane type blood purification device be covered with the radical-trapping material in an amount of 80 to 300% based on the dry weight of the hollow fiber membrane. If the adhesion rate exceeds 300%, the weight of the hollow fiber membrane type blood purification device increases, the advantages of the semi-dry type blood purification device are spoiled, and the handling properties are lost. Moreover, liquid droplets tend to adhere to the inner wall of the container or inside of a sterilization bag at about room temperature (e.g., about 20 to 40°C) at which the blood purification device is stored and distributed, whereby the appearance of the product deteriorates. In particular, if the radical-trapping material is a polyhydric alcohol, the concentration of the radical-trapping material that adheres to the surface and the inside of the membrane locally increases, whereby the viscosity of the radical-trapping material increases. As a result, the coating state of the radical-trapping material tends to become nonuniform, whereby the irradiation protection effect may become insufficient. On the other hand, there is a problem in terms of the production method. Specifically, when the adhesion rate is adjusted in a state of a bundle, the stickiness of the outer surface of the hollow fiber membrane increases, whereby the membranes tend to adhere to each other. The potting material is inhibited to enter, whereby leakage may occur. Moreover, dialysis efficiency may deteriorate when adhesion between the membranes occurs. Therefore, the upper limit of the adhesion rate of the radical-trapping material must be 300% or less, more preferably 200% or less, and particularly preferably 160% or less.

In the present invention, it is preferable that the radical-trapping material be a material which inhibits polyvinylpyrrolidone from crosslinking. It is well known that polyvinylpyrrolidone in the membrane is crosslinked by irradiation to become water-insoluble, thereby elution of polyvinylpyrrolidone is advantageously reduced.
On the other hand, it is also known that if the degree of crosslinking progresses too much, the molecular mobility of polyvinylpyrrolidone in an aqueous medium may be limited, whereby blood coagulation tends to occur when polyvinylpyrrolidone comes into contact with blood. It is possible to prevent polyvinylpyrrolidone from completely crosslinking by partially inhibiting polyvinylpyrrolidone from crosslinking, whereby a decrease in blood compatibility can be suppressed. It is expected that a radical-trapping material generally inhibits a crosslinking reaction by radicals. In the present invention, a radical-trapping material which inhibits polyvinylpyrrolidone from crosslinking is preferably used. Since polyhydric alcohols such as glycerol among the above-mentioned radical-trapping materials clearly inhibit polyvinylpyrrolidone from crosslinking, it is particularly preferable to use polyhydric alcohols as the radical-trapping material. The lower limit of the adhesion rate of the radical-trapping material must be 80% or more from the viewpoint of the protection effect, more preferably 90% or more, and particularly preferably 100% or more.

In the present invention, the adhesion rate of the radical-trapping material is calculated as the total weight of the radical-trapping material to the dry weight of the hollow fiber membrane. The method of measuring the adhesion rate of the radical-trapping material is not particularly limited. When the radical-trapping material is liposoluble, the radical-trapping material is extracted with a solvent which dissolves the radical-trapping material but does not dissolve the membrane material, and the radical-trapping material is quantitatively determined using liquid chromatography, a coloring reagent, or the like. When the radical-trapping material is water-soluble, the radical-trapping material is extracted with warm water or hot water, and is quantitatively determined in the same manner as described above. When the radical-trapping material is an aqueous solution, the water content is also calculated by a water content measurement procedure described later, and the sum of the adhesion rate of the solute and the water content is taken as the adhesion rate.

In the present invention, the hollow fiber membranes must have a water content of not less than 40% and less than 100% based on the dry weight of the hollow fiber membranes in addition to the adhesion rate of the radical-trapping material within the above range. If the water content is not less than 40%, activation of platelets can be suppressed in the initial stage of contact with blood. The detailed reason is not certain, but considered to be as follows. Specifically, polyvinylpyrrolidone is hydrated when the surface of the membranes is moderately wetted, and the membranes exhibit increased wettability in the initial stage of use as compared with almost completely dried membranes, whereby affinity to blood increases. This is a very important feature when it is necessary to use a semi-dry type blood purification device immediately after priming. If the water content is 100% or more, water contained in the pores of the membranes freezes even if water does not exist around the membranes, whereby damage accompanying a change in structure of the hollow fiber membranes tends to occur. If the water content exceeds the equilibrium water content of the membranes, water droplets tend to adhere to the inner wall of the container or the inside of the sterilization bag, whereby the appearance of the product deteriorates.

If the water content is less than 40%, platelets become active in the initial stage of contact with blood, whereby blood compatibility tends to decrease. The reason therefor is considered to be as follows. Specifically, since the molecular mobility of the hydrophilic polymer decreases if the surface of the membranes is dried to a large extent, it takes time for the hydrophilic polymer to get wetted with water and become hydrated. In particular, when the radical-trapping material is a polyhydric alcohol, since the variation in the adhesion rate of the radical-trapping material to the hollow fiber membranes increases due to an increase in viscosity, hollow fiber membranes with an extremely low hydrophilicity tend to be obtained. As a result, blood compatibility tends to decrease. Since a powder or a high-concentration solution of the hydrophilic polymer does not dissolve in water in no time at all, and it takes time to dissolve, it is considered that the

above estimation is appropriate. It is more preferable that the water content be 60% or more.

In the present invention, the method of adjusting the adhesion rate of the radical-trapping material and the water content within the above ranges is not particularly limited. For example, as such method a sequential method, in which a high-concentration solution of the radical-trapping material may be caused to come into contact with the hollow fiber membranes, and the adhesion rate and the water content may be adjusted within specific ranges by causing water to pass through the hollow fiber membranes, can be given. These steps in the above method may obviously be performed in the reverse order. As another example, a method, in which the adhesion rate and the water content may be adjusted in one stage by adjusting the contact time when causing the radical-trapping material solution to come into contact with the membranes, the concentration of the solution, the injection pressure, the flush conditions, and the like, can be given. The latter method preferably has advantages that the process is not so complex and more uniform coating can be achieved since a high-concentration solution need not be used.

These methods may be performed for each hollow fiber membrane during the membrane formation step, or may be performed for a bundle obtained after membrane formation. In the former method, a radical-trapping material solution bath may be provided in the membrane production line, and the hollow fiber membranes may be immersed in the bath. In the latter method, the hollow fiber membrane bundle may be immersed in a radical-trapping material solution bath, or the radical-trapping material solution may be sprayed onto the end of the hollow fiber membrane bundle. Alternatively, a further method includes a method in which the radical-trapping material solution may be caused to pass through the hollow fiber membranes via the openings in the potting portion to the semifabricated product of the hollow fiber membrane type blood purification device (i.e. in a state in which the headers are not attached), or the headers having the fluid inlet port and outlet port connected to the inside of the hollow fiber membranes are attached and then the radical-trapping material solution may be caused to pass through the hollow fiber membranes via the fluid inlet port and outlet port of the header, or the radical-trapping material solution may be caused to pass through the hollow fiber membranes via the fluid inlet port and outlet port of the tubular housing (e.g., dialysate inlet port and outlet port of a hemodialyzer). Any method thereof can be used. Among them, the method to cause the radical-trapping material solution to pass through the hollow fiber membranes via the openings of the semifabricated product of the blood purification device or the fluid inlet port and outlet port of the header of the blood purification device, since even a liquid with a high viscosity can be reliably caused to pass through inside the hollows of the hollow fiber membranes (i.e., contact surface with blood) is preferable.

The adhesion rate of the radical-trapping material and the water content are measured by the following method. 5 g of hollow fiber membranes are collected from the hollow fiber membrane blood purification device as a hollow fiber membrane sample. The weight (A) of the hollow fiber membrane sample before drying is accurately measured. After removing only water using a vacuum dryer, the weight (B) of the hollow fiber membrane sample after drying is measured. The whole hollow fiber membrane sample after drying from which only water has been removed is finely cut. After the addition of 300 ml of pure water, the hollow fiber membrane sample is washed for 60 minutes using an ultrasonic washing device to extract the adhering radical-trapping material (e.g., glycerol). The adhesion amount of the radical-trapping material is determined as follows. The extract obtained by subjecting the cut hollow fiber membrane sample to extraction using the ultrasonic washing device is subjected to quantitative determination by liquid chromatography. A calibration curve is obtained from the peak area of a standard solution, and the amount (C) of the radical-trapping material in the extract is determined using the above calibration curve. Only the cut hollow fiber membrane sample is taken out from the extract and is dried using a vacuum dryer. The weight of the dried hollow fiber membrane sample is measured and taken as the weight (D) of the hollow fiber membranes to which the radical-trapping material and water do not adhere. The water content is calculated by the following equation (1) based on the above measured values, and the adhesion rate of the radical-trapping material is calculated by the following equation (2).

$$\text{Water content (wt\%)} = \{(A-B)/D\}\times 100 \qquad (1)$$

$$\text{Adhesion rate of radical-trapping material (wt\%)} = (C/D)\times 100 \qquad (2)$$

In the present invention, it is preferable that the hollow fiber membranes contain an antioxidant in addition to the radical-trapping material and water. The term "antioxidant" used herein refers to a substance having an antioxidation effect such as sodium pyrosulfite, sodium ascorbate and the like. There can be obtained advantages that the effect of suppressing oxidative deterioration due to oxygen radicals produced during irradiation increases by using the hollow fiber membranes containing the antioxidant.

In the present invention, it is preferable that at least one fluid inlet port or outlet port provided in the hollow fiber

membrane type blood purification device is not plugged. This allows the oxygen concentration in the inner space of the hollow fiber membrane type blood purification device to be more efficiently decreased using an oxygen adjustment method described later. Another fluid inlet port and outlet port (preferably the fluid inlet port and outlet port connected to the hollow fiber membrane inner chamber) other than the above port is plugged so that foreign matter entered into a portion which directly contacts with blood can be advantageously reduced.

In the present invention, it is preferable to adjust the oxygen concentration in the hollow fiber membrane type blood purification device to less than 0.1% when sterilizing the hollow fiber membrane type blood purification device by applying radiation. The effects of the present invention can be increased more highly by combining this method with adjustment of the adhesion rate of the radical-trapping material and the water content according to the present invention. The term "oxygen concentration in the hollow fiber membrane type blood purification device" used herein refers to the oxygen concentration measured in a state in which the hollow fiber membrane type blood purification device is enclosed in a sterilization bag. When the oxygen concentration is the same inside and outside of the hollow fiber membrane type blood purification device, the oxygen concentration is determined by measuring the oxygen concentration in the sterilization bag. When the oxygen concentrations inside and outside of the hollow fiber membrane type blood purification device differ from each other (e.g., when gas impermeable plugs are provided to all fluid inlet port and outlet port), the oxygen concentration is determined by inserting a measurement needle into the plug and measuring the oxygen concentration in the container.

If the oxygen concentration in the hollow fiber membrane type blood purification device is low, oxidative decomposition due to cutting of the polymer main chain caused by oxygen radicals produced due to irradiation can be suppressed, whereby elution of polyvinylpyrrolidone can be suppressed. On the other hand, when the oxygen concentration in the hollow fiber membrane type blood purification device is high same as that of the atmosphere, if the adhesion rate of the radical-trapping material and the water content are not within the ranges specified in the present invention, the membranes cannot be sufficiently protected from irradiation. As a result, polyvinylpyrrolidone forming the membranes is reversely crosslinked and modified due to a decrease in oxygen concentration in the atmosphere of the hollow fiber membrane, whereby blood compatibility decreases to a large extent. On the other hand, if the adhesion rate of the radical-trapping material and the water content are within the ranges specified in the present invention, excessive crosslinking of polyvinylpyrrolidone is suppressed even if the oxygen concentration in the hollow fiber membrane type blood purification device is almost equal to that of the atmosphere, whereby excellent blood compatibility can be achieved. According to the present invention, a blood purification device which stably exhibits excellent blood compatibility for a long time can be provided without strictly controlling the oxygen concentration.

In the present invention, when the adhesion rate of the radical-trapping material is 80 to 300% and the water content is not less than 40% and less than 100%, it is important and more preferable from the viewpoint of reducing the amount of elution that the oxygen concentration in the atmosphere of the hollow fiber membrane when subjecting to radiation sterilization be less than 0.1%. If the oxygen concentration is less than 0.1%, oxidative decomposition ofpolyvinylpyrrolidone forming the hollow fiber membranes can be further suppressed. Therefore, elution of polyvinylpyrrolidone can be further suppressed, and blood compatibility thus can be improved. It takes time to reduce the oxygen concentration to a predetermined concentration of less than 0.01 % in a actual process, and it is inevitable to use a sterilization bag which does not allow gas to permeate through at all, whereby the production cost increases. Moreover, polyvinylpyrrolidone is further crosslinked and modified when reducing the oxygen concentration to a large extent, whereby blood compatibility may deteriorate. Therefore, the oxygen concentration is more preferably not less than 0.01 % and less than 0.1 %.

The hollow fiber membranes, the potting material, the container, the header, and the like are less affected by irradiation under said condition as compared with the case where the oxygen concentration is high. Moreover, coloring and odors can advantageously be reduced.

In the present invention, the hollow fiber membrane type blood purification device in the above state is packed in the sterilization bag. The oxygen concentrations in the sterilization bag and the hollow fiber membrane type blood purification device are adjusted by replacing the air with an inert gas such as $N_2$, He, $CO_2$, Ar, or He, or by using an oxygen absorber. Note that the oxygen concentration adjustment method is not limited thereto. When using an oxygen absorber, it is preferable to use an oxygen absorber which does not produce other gas components during absorbing oxygen or does not lose its activity due to irradiation. For example, an oxygen absorber is preferable of which the reaction rate is controlled by a catalyst containing an active metal(s) as the main component and. Examples of the active metal include iron, zinc, copper, tin, and the like. In particular, an oxygen absorber which contains active iron oxide as the main component is preferable. An example of a commercial available product of such an oxygen absorber is Ageless (registered trademark) manufactured by Mitsubishi Gas Chemical Company Inc. Note that the oxygen absorber is not limited thereto.

The term "radiation sterilization" used in the present invention refers to sterilization using electron beams, $\gamma$-rays, or the like. The irradiation dose is preferably 5 to 50 kGy, more preferably 15 to 30 kGy, and particularly preferably about 25 kGy.

EXAMPLES

The present invention is described below in more detail by way of examples.
Note that the present invention is not limited to the following examples.

(Measurement of oxygen concentration)

The oxygen concentration was measured before and after radiation sterilization using a trace oxygen analyzer "RO-102" manufactured by Iijima Electronics Corporation in a state in which the hollow fiber membrane type blood purification device was enclosed in a sterilization bag.

(Method of measuring lactate dehydrogenase (LDH) as index of platelet activation)

The blood purification device was disassembled to sample the hollow fiber membranes. The both ends of the sampled hollow fiber membranes were processed with silicone so that the effective length was 15 cm and the inner membrane surface area was 50 $mm^2$ to form a mini module. The mini module was washed by flowing 10 ml of a physiological saline solution ("Otsuka Normal Saline" manufactured by Otsuka Pharmaceutical Co., Ltd.) through the insides of the hollow fiber membranes (hereinafter referred to as "priming"). 7 ml of heparinized human blood was then placed in a syringe pump, and was caused to pass through the inside of mini module at a rate of 1.44ml/min. The inside and the outside of the mini module were respectively washed with 10 ml of a physiological saline solution. Half of the hollow fiber membranes with a length of 14cm were collected from the washed mini module. The hollow fiber membranes were then cut finely and placed in an LDH measurement conical tube to obtain a measurement sample. 0.5 ml of a 0.5 vol% TritonX-100/PBS solution obtained by dissolving Triton X-100 (manufactured by Nakalai Tesque, Inc.) in a phosphate buffer solution (PBS) (manufactured by Wako Pure Chemical Industries, Ltd.) was added to the LDH measurement conical tube. An ultrasonic treatment was conducted for 60 minutes to destroy the cells (mainly platelets) adhering to the hollow fiber membranes, and the LDH in the cells was extracted. 0.05 ml of the extract was sampled, and added with 2.7 ml of a 0.6 mM sodium pyruvate solution and 0.3 ml of a nicotinamide adenine dinucleotide (NADH) solution (1.277 mg/ml) to react. 0.5 ml of the reaction mixture was immediately sampled to measure the absorbance at 340 nm. After allowing the residue of the reaction mixture to react at 37°C for one hour, the absorbance at a 340 nm was measured. A decrease in absorbance from the time immediately after the beginning of the reaction was determined. The absorbance was also measured for the membranes which were not reacted with blood. The difference in absorbance was calculated by the following equation (3), and was divided by the measured membrane area. In this method, the larger the decrease rate, the higher the LDH activity, more specifically it means activation of platelets is larger.

$$\Delta 340 \text{ nm} = [(\text{absorbance of sample immediately after reaction beginning} - \text{absorbance of sample after 60 minutes}) - (\text{absorbance of blank immediately after reaction beginning} - \text{absorbance of blank after 60 minutes})] / \text{inner surface area of hollow fiber membrane} \quad (3)$$

(Method of measuring elution amount of PVP)

The blood side and the dialysate side of the blood purification device were respectively washed with 1 liter or more of injection water ("Otsuka Distilled Water" manufactured by Otsuka Pharmaceutical Ltd.) sufficiently. The liquid was sufficiently removed by injecting compressed air. Injection water ("Otsuka Distilled Water" manufactured by Otsuka Pharmaceutical Ltd.) heated at 70°C was circulated through the blood side at a rate of 200 ml/min for one hour in a state in which the dialysate side of the blood purification device is sealed. After one hour, the extract was filtered through a filter with a pore size of 0.45 μm. The PVP concentration in the filtrate was measured using an HPLC ("LC-10AD/SPD-10AV" manufactured by Shimadzu Corporation). The HPLC conditions were as follows.
Column: Shoudex Asahipak GF-710HQ
Mobile phase: 50 mM NaCl aqueous solution
Flow rate: 1.0 ml/min
Temperature: 30°C
Detection: 220 nm

Injection: 50 micro litters

(Method of measuring absorption height of dye aqueous solution)

The blood side and the dialysate side of the blood purification device were respectively washed with 1 liter or more of injection water (Otsuka Distilled Water manufactured by Otsuka Pharmaceutical Ltd.) sufficiently. The liquid was sufficiently removed by injecting compressed air. The blood purification device was then disassembled, and the hollow fiber membranes were taken out. Injection water (Japanese Pharmacopoeia) remaining in the hollow fiber membranes were sufficiently removed by air-flashing using a syringe. The hollow fiber membranes were vertically suspended in a 1% Congolese red (manufactured by Wako Pure Chemical Co., Ltd.) aqueous solution so that the end portion is immersed by 2 mm. The liquid surface of the solution is taken as 0 mm in the absorption height. 30 seconds after beginning of immersion, the absorption height of the 1% Congolese red aqueous solution was read accurately. The measurement was conducted at room temperature (25°C). Twenty hollow fiber membranes per each sample were picked up and measured. The absorption height of the dye aqueous solution is a value which mainly indicates the hydrophilicity of the inner surface of the hollow fiber membrane. The higher the absorption height, the higher the hydrophilicity is. The larger the variation in the absorption height, the larger the difference in hydrophilicity between the hollow fiber membranes is.

Example 1

A homogeneous spinning solution was prepared from 17 parts by weight of PSf ("P-1700" manufactured by Solvay Advanced Polymers, K.K.), 4 parts by weight of PVP ("K-90" manufactured by ISP), and 79 parts by weight of dimethylacetamide (hereinafter referred to as "DMAC"). A 42% DMAC aqueous solution was used as a hollow-making inner solution. The spinning solution and the DMAC aqueous solution were discharged from a spinneret. The amounts of the spinning solution and the hollow-making inner solution discharged were adjusted so that the thickness and the inner diameter of the membrane after drying were 45 μm and 185 μm, respectively.
The discharged spinning solution was immersed in a coagulation bath (60°C) which was provided at a position 50 cm below the spinneret and contained water. The spinning solution was subjected to coagulation and washing with water at 30 m/min, and was dried at 160°C in a dryer. The polysulfone-based hollow fiber membrane was then crimped and winded.
A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 61.1%) was injected into the hollow fiber membranes from the open end for five seconds. A header cap was then attached to each end of the container. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 146%, and the water content was 93.4%.
The performance measurement results are shown in Table 1 (Table 1.1).

Example 2

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 58.7%) was injected into the hollow fiber membranes from the open end for 2.9 seconds. A header cap was then attached to each end of the container. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 85.6%, and the water content was 60.2%.
The performance measurement results are shown in Table 1 (Table 1.1).

Example 3

Polysulfone-based hollow fiber membranes produced in the same manner as in Example 1 were winded. A

plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 60.7%) was injected into the hollow fiber membranes from the open end for 4.5 seconds. A header cap was then attached to each end of the container. After plugging blood outlet and inlet nozzles, the product was placed in a sterilization bag. After adjusting the oxygen concentration to 0.05%, γ-rays were applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 140.2%, and the water content was 90.8%. The performance measurement results are shown in Table 1 (Table 1.1).

Example 4

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. The product was processed in the same manner as in Example 3. A header cap was then attached to each end of the container. After plugging blood outlet and inlet nozzles, the product was placed in a sterilization bag. After adjusting the oxygen concentration to 0.10%, γ-rays were applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 140.4%, and the water content was 90.6%. The performance measurement results are shown in Table 1 (Table 1.1).

Example 5

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 58.5%) was injected into the hollow fiber membranes from the open end for 4.7 seconds. A header cap was then attached to each end of the container. After plugging blood outlet and inlet nozzles, the product was placed in a sterilization bag. After adjusting the oxygen concentration to 0.04%, γ-rays were applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 81.9%, and the water content was 58.1%.
The performance measurement results are shown in Table 1 (Table 1.1).

Example 6

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 86.6%) was injected into the hollow fiber membranes from the open end for 6.4 seconds. A header cap was then attached to each end of the container. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 275.1%, and the water content was 42.5%. The performance measurement results are shown in Table 1 (Table 1.1).

Example 7

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 76.2%) was injected into the hollow fiber membranes from the open end for 7.6 seconds. A

header cap was then attached to each end of the container. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 286.3%, and the water content was 89.4%. The performance measurement results are shown in Table 1 (Table 1.1).

Example 8

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 55.7%) was injected into the hollow fiber membranes from the open end for 2.0 seconds. A header cap was then attached to each end of the container. After plugging blood outlet and inlet nozzles, the product was placed in a sterilization bag. After adjusting the oxygen concentration to 0.04%, γ-rays were applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 55.6%, and the water content was 44.3%.
The performance measurement results are shown in Table 1 (Table 1.2).

Example 9

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A polyethylene glycol (PEG600 manufactured by Katayama Chemical Industries Co., Ltd.) aqueous solution (concentration: 61.0%) was injected into the hollow fiber membranes from the open end for 4.5 seconds. A header cap was then attached to each end of the container. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.
In this hollow fiber membrane type blood purification device, the adhesion rate of polyethylene glycol (radical-trapping material) was 142.4%, and the water content was 91.2%. The performance measurement results are shown in Table 1 (Table 1.2).

Example 10

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 60.5%) was injected into the hollow fiber membranes from the open end for 4.5 seconds. A header cap was then attached to each end of the container. After plugging blood outlet and inlet nozzles, the product was placed in a sterilization bag. After adjusting the oxygen concentration to 10.4%, γ-rays were applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 143.3%, and the water content was 93.5%. The performance measurement results are shown in Table 1 (Table 1.2).

Example 11

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 61.1%) was injected into the hollow fiber membranes from the open end for 4.4 seconds. A header cap was then attached to each end of the container. After plugging blood outlet and inlet nozzles, the product was placed in a sterilization bag. After adjusting the oxygen concentration to 5.81 %, γ-rays were applied to the product

at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 142.5%, and the water content was 90.9%. The performance measurement results are shown in Table 1 (Table 1.2).

Example 12

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 60.8%) was injected into the hollow fiber membranes from the open end for 4.4 seconds. A header cap was then attached to each end of the container. After plugging blood outlet and inlet nozzles, the product was placed in a sterilization bag. After adjusting the oxygen concentration to 0.93%, γ-rays were applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 141.6%, and the water content was 91.4%. The performance measurement results are shown in Table 1 (Table 1.2).

Example 13

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 60.7%) was injected into the hollow fiber membranes from the open end for 4.5 seconds. A header cap was then attached to each end of the container. After plugging blood outlet and inlet nozzles, the product was placed in a sterilization bag. After adjusting the oxygen concentration to 0.52%, γ-rays were applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 144.3%, and the water content was 93.3%. The performance measurement results are shown in Table 1 (Table 1.2).
Table 1-1

| | Example | | | | | | |
|---|---|---|---|---|---|---|---|
| | 1 | 2 | 3 | 4 | 5 | 6 | 7 |
| Radical-trapping material | glycerol | glycerol | glycerol | glycerol | glycerol | glycerol | glycerol |
| Adhesion rate of radical-trapping material (wt%) | 146.6 | 85.6 | 140.2 | 140.4 | 81.9 | 275.1 | 286.3 |
| Water content (wt%) | 93.4 | 60.2 | 90.8 | 90.6 | 58.1 | 42.5 | 89.4 |
| Oxygen concentration (%) | 21.9 | 21.9 | 0.05 | 0.10 | 0.04 | 21.9 | 21.9 |
| LDH (Δabs/hr/$m^2$) | 20.7 | 22.8 | 12.5 | 18.7 | 13.8 | 18.9 | 20.1 |
| PVP elution amount (mg) | 3.5 | 3.8 | 0.26 | 3.6 | 0.27 | 2.9 | 3.2 |

(continued)

| | Example | | | | | | |
|---|---|---|---|---|---|---|---|
| | 1 | 2 | 3 | 4 | 5 | 6 | 7 |
| Absorption height of dye aqueous solution (average value±standard deviation) (mm) | 160.6 ±5.1 | 98.4 ±7.8 | 103.3 ±5.2 | 106.2 ±5.0 | 98.0 ±7.9 | 96.5 ±7.2 | 105.4 ±5.1 |
| Appearance ofpropduct | good | good | good | good | good | good | good |

Table 1-2

| | Example | | | | | |
|---|---|---|---|---|---|---|
| | 8 | 9 | 10 | 11 | 12 | 13 |
| Radical-trapping material | glycerol | PEG* | glycerol | glycerol | glycerol | glycerol |
| Adhesion rate of radical-trapping material (wt%) | 55.6 | 142.4 | 143.3 | 142.5 | 141.6 | 144.3 |
| Water content (wt%) | 44.3 | 91.2 | 93.5 | 90.9 | 91.4 | 93.3 |
| Oxygen concentration (%) | 0.04 | 21.9 | 10.4 | 5.81 | 0.93 | 0.52 |
| LDH (Δabs/hr/$m^2$) | 15.4 | 20.2 | 19.7 | 18.2 | 21.6 | 19.5 |
| PVP elution amount (mg) | 0.36 | 3.6 | 3.6 | 3.4 | 3.2 | 3.3 |
| Absorption height of dye aqueous solution (average value±standard deviation) (mm) | 98.0 ±7.8 | 101.1 ±5.1 | 103.5 ±5.2 | 102.6 ±5.0 | 105.3 ±5.0 | 105.6 ±5.2 |
| Appearance of product | good | good | good | good | good | good |
| * PEG: Polyethylene glycol | | | | | | |

Comparative Example 1

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 86.9%) was injected into the hollow fiber membranes from the open end for 3.5 seconds. A header cap was then attached to each end of the container. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 156.3%, and the water content was 23.5%. The performance measurement results are shown in Table 2. It is considered that the LDH activity increased since glycerol as the radical-trapping material was not uniformly applied to the hollow fiber membranes.

Comparative Example 2

Polysulfone-based hollow fiber membranes produced in the same manner as in Example 1 were winded. A plastic tubular container designed so that the effective membrane area was 1.5 $m^2$ was loaded with a bundle of winded

10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 90.4%) was injected into the hollow fiber membranes from the open end for 7.2 seconds. A header cap was then attached to each end of the container. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 315.2%, and the water content was 33.4%. The performance measurement results are shown in Table 2. The LDH activity was as high as in Comparative Example 1. This indicates that glycerol was not uniformly applied to the hollow fiber membranes.

Comparative Example 3

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 31.5%) was injected into the hollow fiber membranes from the open end for 2.6 seconds. A header cap was then attached to each end of the container. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 42.5%, and the water content was 92.5%.
The performance measurement results are shown in Table 2. A large number of water droplets adhered to the inside of the hollow fiber membrane type blood purification device and the sterilization bag.

Comparative Example 4

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A header cap was attached to each end of the container without applying a radical-trapping material and water. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.
The performance measurement results of this hollow fiber membrane type blood purification device are shown in Table 2.

Comparative Example 5

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A header cap was attached to each end of the container without applying a radical-trapping material and water. After plugging blood outlet and inlet nozzles, the product was placed in a sterilization bag. After adjusting the oxygen concentration to 0.05%, γ-rays were applied to the product at a dose of 25 kGy to obtain a hollow fiber membrane type blood purification device having an effective membrane area of 1.5 $m^2$.
The performance measurement results of this hollow fiber membrane type blood purification device are shown in Table 2.

Comparative Example 6

Polysulfone-based hollow fiber membranes produced in the same manner as in Example 1 were winded. A plastic tubular container designed so that the effective membrane area was 1.5 $m^2$ was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 26.8%) was injected into the hollow fiber membranes from the open end for 0.5 seconds. A header cap was then attached to each end of the container. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.

In this hollow fiber membrane type blood purification device, the adhesion rate of glycerol (radical-trapping material) was 12.1%, and the water content was 33.0%. The performance measurement results are shown in Table 2. It is considered that the LDH activity increased since the amount of glycerol as the radical-trapping material was small with respect to the hollow fiber membranes, whereby the inner surfaces of the hollow fiber membranes were not uniformly coated. The elution amount of PVP was also increased.

Comparative Example 7

Polysulfone-based hollow fiber membranes produced in the same manner as in Example 1 were winded. A plastic tubular container designed so that the effective membrane area was 1.5 m2 was loaded with a bundle of winded 10,000 hollow fiber membranes. Each end of the bundle was secured using a urethane resin and was cut to form open ends of the hollow fiber membranes. A glycerol (special grade, manufactured by Wako Pure Chemical Co., Ltd.) aqueous solution (concentration: 73.9%) was injected into the hollow fiber membranes from the open end for 8.5 seconds. A header cap was then attached to each end of the container. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 321.5%, and the water content was 113.4%. The performance measurement results are shown in Table 2. It is considered that the LDH activity increased since the hollow fiber membranes were not uniformly coated with glycerol. A large number of water droplets adhered to the inside of the hollow fiber membrane type blood purification device and the sterilization bag.

Comparative Example 8

Polysulfone-based hollow fiber membranes produced in the same manner as in Example 1 were winded. A bundle of 10,000 hollow fiber membranes was caused to come into contact with a glycerol aqueous solution (concentration: 80 wt%). The product was dried at 60°C. A plastic tubular container designed so that the effective membrane area was 1.5 $m^2$ was loaded with the hollow fiber membrane bundle. Each end of the bundle was secured using a urethane resin and was cut to form open ends of the hollow fiber membranes. A header cap was then attached to each end of the container. After plugging blood outlet and inlet nozzles, the product was placed in a sterilization bag. γ-rays were then applied to the product at a dose of 25 kGy to obtain a hollow fiber membrane type blood purification device having an effective membrane area of 1.5 $m^2$.
In this hollow fiber membrane type blood purification device, the adhesion rate of glycerol (radical-trapping material) was 312.2%, and the water content was 35.0%. The performance measurement results are shown in Table 2. It is considered that the LDH activity increased since glycerol as a radical-trapping material was not uniformly applied to the hollow fiber membranes. Moreover, the hollow fiber membranes adhered to each other.
Table 2

| | Comparative example | | | | | | | |
|---|---|---|---|---|---|---|---|---|
| | 1 | 2 | 3 | 4 | 5 | 6 | 7 | 8 |
| Radical-trapping material | glycerol | glycerol | glycerol | - | - | glycerol | glycerol | glycerol |
| Adhesion rate of radical-trapping material (wt%) | 156.3 | 315.2 | 42.5 | - | - | 12.1 | 321.5 | 312.2 |
| Water content (wt%) | 23.5 | 33.4 | 92.5 | - | - | 33.0 | 113.4 | 35.0 |
| Oxygen concentration (%) | 21.9 | 21.9 | 21.9 | 21.9 | 0.05 | 21.9 | 21.9 | 21.9 |
| LDH ($\Delta$abs/hr/$m^2$) | 63.9 | 64.9 | 65.1 | 63.6 | 83.4 | 61.6 | 18.5 | 65.7 |

(continued)

| | Comparative example | | | | | | | |
|---|---|---|---|---|---|---|---|---|
| | 1 | 2 | 3 | 4 | 5 | 6 | 7 | 8 |
| PVP elution amount (mg) | 17.9 | 17.5 | 19.9 | 25.8 | 0.71 | 23.3 | 3.3 | 17.0 |
| Absorption height of dye aqueous solution (average value±standard deviation) (mm) | 86.5 ±10.2 | 89.4 ±12.3 | 98.0 ±7.7 | 82.0 ±10.1 | 82.3 ±10.1 | 85.0 ±12.1 | 105.4 ±5.1 | 70.1 ±19.2 |
| Appearance of product | good | *1 | *2 | good | good | good | *3 | *4 |
| *1: Droplets of glycerol were observed in container and sterilization bag<br>*2: Water droplets were observed in container and sterilization bag<br>*3: A large number of water droplets were observed in container and sterilization bag<br>*4: Hollow fiber membranes partially adhered each other | | | | | | | | |

In Tables 1 and 2, the "appearance of product" indicates the presence or absence of water droplets or the radical-trapping material adhering to the bag, or the presence or absence of adhesion between the hollow fiber membranes. A case where no abnormal change was observed was indicated as "Good".

INDUSTRIAL APPLICABILITY

The hollow fiber membrane type blood purification device according to the present invention is so-called "semi-dry" and has a reduced weight and excellent handling properties, exhibits a small amount of elution of polyvinylpyrrolidone, and exhibits excellent blood compatibility. Therefore, the blood purification device according to the present invention may be used for a medical treatment of various diseases without impairing safety.
Since the blood purification device according to the present invention exhibits excellent safety and compatibility with the living body, the blood purification device according to the present invention is suitably used especially in the field of hemodialysis and hemofiltration where therapy continues for a long time at a high frequency.

**Claims**

1. A hollow fiber membrane type blood purification device comprising a container loaded with a bundle of hollow fiber membranes which are formed of a polysulfone-based resin and polyvinylpyrrolidone, wherein the gaps between each ends of the hollow fiber membrane bundle and the container are held by a potting material to form a hollow fiber membrane inner chamber and a hollow fiber membrane outer chamber, the hollow fiber membrane type blood purification device has fluid inlet and outlet ports connected to the hollow fiber membrane inner chamber and fluid inlet and outlet ports connected to the hollow fiber membrane outer chamber, and the hollow fiber membranes have 80 to 300% of adhesion rate of a radical-trapping material to the dry weight of the hollow fiber membranes and not less than 40% and less than 100% of a water content, and the hollow fiber membranes are sterilized by radiation.

2. The hollow fiber membrane type blood purification device according to claim 1, wherein the radical-trapping material is a material which inhibits polyvinylpyrrolidone from crosslinking.

3. The hollow fiber membrane type blood purification device according to claim 1 or 2, wherein the radical-trapping material is a polyhydric alcohol.

4. The hollow fiber membrane type blood purification device according to any one of claims 1 to 3, wherein the radical-trapping material is glycerol.

5. The hollow fiber membrane type blood purification device according to any one of claims 1 to 4, wherein the adhesion rate of the radical-trapping material is 100 to 160%.

6. The hollow fiber membrane type blood purification device according to any one of claims 1 to 5, wherein the hollow fiber membranes are treated with the radical-trapping material by

a) causing a radical-trapping material solution to pass through the hollow fiber membranes from opening thereof after the gaps between the ends of the hollow fiber membrane bundle and the container have been held by the potting material and before headers have been provided, or
b) causing a radical-trapping material solution to pass through the hollow fiber membranes using fluid inlet and outlet ports connected to the inside of the hollow fiber membranes of the blood purification device after headers respectively having a fluid inlet port and outlet port have been equipped.

7. The hollow fiber membrane type blood purification device according to any one of claims 1 to 6, wherein the hollow fiber membranes have been subjected to γ-ray sterilization in a state in which the oxygen concentration in the hollow fiber membrane type blood purification device is less than 0.1 %.

8. The hollow fiber membrane type blood purification device according to any one of claims 1 to 7, wherein the amount of polyvinylpyrrolidone eluted from the hollow fiber membranes is not more than 5 mg per 1.5 $m^2$ of the inner surface area of the hollow fiber membranes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S5523620 B
- JP 2000288085 A
- JP H06285162 A
- JP 2003245526 A
- JP 2001170167 A
- JP 2001170172 A
- JP 2000196318 A